# EUROPEAN PATENT SPECIFICATION

(11) **EP 3 218 021 B1**
(45) Date of publication and mention of the grant of the patent: **11.03.2020**
(21) Application number: 15798630.8
(22) Date of filing: 11.11.2015
(51) Int. Cl.: A61L 27/18, A61L 27/34, A61L 27/54, A61L 27/58

(54) **COMPOSITIONS FOR USE IN A METHOD FOR THE PREVENTION AND/OR REDUCTION OF SCARRING**
ZUSAMMENSETZUNGEN ZUR VERWENDUNG IN EINER METHODE ZUR VORBEUGUNG UND/ODER REDUKTION VON NARBENBILDUNG
COMPOSITIONS POUR UTILISATION DANS UNE METHODE POUR ÉVITER ET/OU RÉDUIRE LA FORMATION DE CICATRICES

(30) Priority: 11.11.2014 US 201462077953 P; 13.04.2015 US 201562146521 P
(43) Date of publication of application: 20.09.2017
(73) Proprietor: Duke University, Durham, North Carolina 27705 (US)
(72) Inventor: LEVINSON, Howard, Durham, NC 27710 (US); LEONG, Kam W., Durham, NC 27708 (US); LORDEN, Elizabeth R., Durham, NC 27708 (US); MILLER, Kyle J., Durham, NC 27705 (US)
(74) Representative: De Clercq & Partners
(86) International application number: PCT/US2015/060199
(87) International publication number: WO 2016/077480

(56) References cited:
- WO-A1-2010/042651
- WO-A1-2013/018021
- WO-A1-2014/172465
- WO-A2-2008/094971
- BONG SEOK JANG ET AL: "Fibroblast culture on poly(L-lactide-co-epsilon-caprolactone) an electrospun nanofiber sheet", MACROMOLECULAR RESEARCH, vol. 20, no. 12, 1 December 2012 (2012-12-01), pages 1234-1242, XP55236952, KR ISSN: 1598-5032, DOI: 10.1007/s13233-012-0180-5
- SANGWON CHUNG ET AL: "Nanofibrous scaffolds electrospun from elastomeric biodegradable poly(L-lactide-co-[epsilon]-caprolactone) copolymer; Nanofiberous scaffolds electrospun from elastomeric biodegradable PLCL copolymer", BIOMEDICAL MATERIALS, INSTITUTE OF PHYSICS PUBLISHING, BRISTOL, GB, vol. 4, no. 1, 1 February 2009 (2009-02-01), page 15019, XP020157729, ISSN: 1748-605X
- NING-HUA LIU ET AL: "Electrospinning of poly ([epsilon]-caprolactone-co-lactide)/Pluron ic blended scaffolds for skin tissue engineering", JOURNAL OF MATERIALS SCIENCE, vol. 49, no. 20, 15 July 2014 (2014-07-15) , pages 7253-7262, XP055236946, Dordrecht ISSN: 0022-2461, DOI: 10.1007/s10853-014-8432-8

## Description

### BACKGROUND OF THE INVENTION

### Field of the Invention

The scope of the invention is defined by the claims. Any references in the description to methods of treatment refer to the scaffold for use in a method for treatment of the human body by therapy.

The present disclosure provides biocompatible electrospun scaffolds useful in the methods of the disclosure.

### Description of Related Art

Dermal scarring affects more than 80 million people worldwide annually. For example, over 4.4 million people are injured in motor vehicle accidents, thousands of soldiers are wounded in military exercises, and over 2.4 million patients are burned. The World Health Organization states that "there is no doubt that the social and medical costs of bums are significant. Economic impact of burns includes lost wages, and the costs related to deformities from burns, in terms of emotional trauma and lost skill."

Following dermal injury, resident fibroblasts in the wound bed react to inflammatory cytokines and mechanical tension by differentiating into contractile myofibroblasts. The two identifying features of a myofibroblast are (1) the presence of a contractile apparatus, similar to that of smooth muscle cells, and (2) the neo-expression of the actin isoform found in vascular smooth muscle cells, α-smooth muscle actin (αSMA). When de novo αSMA is incorporated into stress fibers, myofibroblasts gain the capacity to produce strong contractile force and physically contract the wound bed. While this process is critical to successful wound repair and leads to the positive outcome of reducing the wound surface area, its success relies on a massive coordinated wave of myofibroblast apoptosis upon wound closure. Myofibroblast apoptosis can be detected 12 days after wounding, and is believed to peak at day 20 during normal scar formation. However, this wave of myofibroblast apoptosis does not occur during fibrotic phenomena such as scar contracture. Rather, these cells persist in the wound bed and continue to contract the wound and secrete cytokines promoting prolonged cellular proliferation.

During scar contraction, myofibroblasts persist in the granulation tissue and pathologically contract the scar. Myofibroblasts are thought to enter this pathologic scarring stage through the generation of a positive feedback loop, in which: (1) resident fibroblasts are induced to myofibroblast phenotype by a combination of mechanical forces and inflammatory cytokines; (2) once they enter the myofibroblast lineage they become contractile; (3) in the contractile state they exert mechanical forces on their surrounding environment and secrete cytokines into the wound bed. Through this process, myofibroblasts contract the scar and induce surrounding fibroblasts to further differentiate into the myofibroblast cell type.

In severe burns (about 28,000 patients/year in the United States), the incidence of hypertrophic scar (HSc) is 40-70 %. HSc are firm, raised, red, itchy scars that are disfiguring and can have a severe impact on quality of life. 70% of HSc occur across joints or other areas of high tension in the body, resulting in a scar contractures that restrict range-of-motion.

Severe burn injuries are typically treated using a collagen-based, biodegradable scaffold, followed by the placement of a skin graft. Although these scaffolds have been used in the clinic for about 30 years, they remain unable to prevent HSc formation. To date, there are no effective measures to prevent HSc. As a result, patients with scar contractures must undergo additional surgical procedures to restore form and function.

Current preventative therapies for scar contracture are ineffective, and patients requiring intervention undergo at least four corrective surgeries on average. HSc develops during the first 4-8 weeks following injury and continues to mature and contract throughout the remodeling phase of repair for as long as six months post trauma; however, commercially available bioengineered skin equivalents (BSE) degrade and are remodeled in the wound bed within 1-4 weeks. There are currently three BSEs on the market which are approved for third degree burn treatment: Epicel, TransCyte, and Integra. Of these, Integra is the most widely used for treatment due to its cost and ease of use. Integra Dermal Regeneration Template (Integra Life Sciences, Plainsboro, NJ) is a bovine collagen-based lyophilized matrix covered by a silicone dressing. The biologic layer remains in the wound bed, while the silicone layer is removed prior to application of skin graft. Previous work studying collagen-based scaffolds for dermal regeneration has shown that prolonging the half-life of the material in the wound bed has a profound impact on minimizing wound contraction; however, the longest scaffold half-life tested in these studies was 2- 4 weeks. It is hypothesized that to improve mitigation of HSc, the scaffold should be present in the wound bed throughout the remodeling phase of repair when HSc occurs.

The main reason for HSc contractures is rapid degradation of collagen based scaffolds. Previous work studying collagen-based scaffolds for dermal regeneration has shown that prolonging the half-life of the materials has a profound impact on minimizing scar contraction. However, the longest scaffold half-life tested in these studies was 2-4 weeks.
It is hypothesized that scaffolds must be present in the wound bed for at least 6 months to prevent HSc.

WO 2010/042651 describes an electrospun scaffold comprising 2 layers of aligned polymer fibers, each comprising PLCL.

The two most important principles to developing a scaffold to prevent HSc contraction are: the scaffold should be bioresorbable, last for 6-18 months *in vivo,* contain topographical cues akin to unwounded skin, and contain mechanical properties akin to unwounded skin.

### SUMMARY OF THE INVENTION

The present disclosure addresses these shortcomings by utilizing a novel bioengineered scaffold for use in a method of preventing and/or reducing HSc.

The scope of the invention is defined by the claims. Any references in the description to methods of treatment refer to the scaffolds for use in a method for treatment of the human body by therapy.

One aspect of the present disclosure provides a biocompatible scaffold comprising poly(l-lactide-co-ε-caprolactone) (PLCL). The biocompatible scaffold of the disclosure is electrospun. In some embodiments, the biocompatible electrospun scaffold comprises a 50:50 mixture of poly(lactic acid) (PLA) and poly(ε-caprolactone) (PCL). The inventors have discovered that the scaffolds of the disclosure beneficially persist throughout the remodeling phase of repair (e.g., about 6 to 18 months), exhibit minimal foreign body response, and display elastomeric properties appropriate for placement and good incorporation beneath skin graft. In addition, the scaffolds of the disclosure have the ability to prevent cell-mediated substrate contraction and decrease fibroblast-to-myofibroblast transformation. In addition, studies conducted *in vivo* in an immune-competent murine HSc contraction model demonstrated that the scaffolds of the disclosure have improved prevention of HSc contraction as compared to the clinical standard of care.

The biocompatible scaffold of the disclosure is coated with collagen I to improve host-scaffold interactions.

In some embodiments, the biocompatible scaffold of the disclosure is combined with one or more additional compounds. In one embodiment, the additional compound comprises an anti-scarring compound. In some embodiments, the anti-scarring compound comprises a statin. In other embodiments, the anti-scarring compound comprises losartan. In some embodiments, the anti-scarring compound comprises a micro inhibitory RNA (miR), growth factor, peptide, and/or antibody.

In yet another embodiment, the biocompatible scaffold is combined with a pro-healing compound(s). In some embodiments, the pro-healing compound comprises a miR, growth factor, peptide, or antibody.

In yet another embodiment, the biocompatible scaffold is combined with an anti-inflammatory compound(s). In some embodiments, anti-inflammatory compound comprises a miR, growth factor, peptide, or antibody.

Also disclosed is a method of preventing and/or reducing HSc in a subject comprising, consisting of, or consisting essentially of implanting a biocompatible electrospun scaffold in a wound of the subject.

Another aspect of the present disclosure provides a method of preventing and/or reducing HSc of a subject comprising, consisting of, or consisting essentially of implanting a biocompatible electrospun scaffold in the wound of a subject.

In one embodiment, the scaffold is implanted subcutaneously.

In one embodiment, the wound comprises a chronic wound. In some embodiments, the chronic wound comprises a venous stasis ulcer or a diabetic foot ulcer.

In another embodiment, the wound comprises fibrosis following trauma, thermal injury or radiation damage.

In another embodiment, the wound comprises a surgical wound. In some embodiments, the surgical wound comprises a wound following aesthetic surgery, hernia repair, dura repair, orbital floor repair, breast reconstruction, urological repair, gynecological repair, and combinations thereof.

### BRIEF DESCRIPTION OF THE DRAWINGS

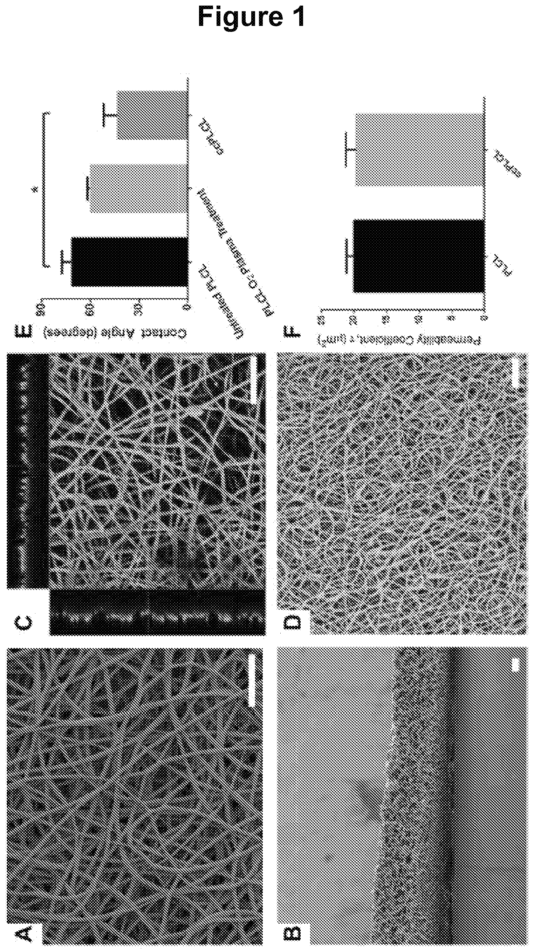

**Figure 1** shows characterization of PLCL Scaffolds. Scaffolds were imaged using SEM: fiber diameter values were measured from top-view images **(A)** while scaffold overall thickness was measured via cross-sectional imaging **(B).** Scaffolds were covalently coated with Bovine type 1 collagen, collagen was stained using AlexaFluor488 and imaged via confocal microscopy **(C)** to analyze coaling efficiency. Collagen coating did not fill pores or alter scaffold topography when imaged on confocal **(C)** or SEM **(D).** Contact angle was analyzed on PLCL films, following collagen coating films were significantly more hydrophilic than untreated PLCL films. Permeability analysis was carried out on PLCL scaffolds 110 µm thick, the Darcy coefficient of permeability was not significantly different between untreated PLCL and ccPLCL. Scale bar is set to 100 µm for all images.

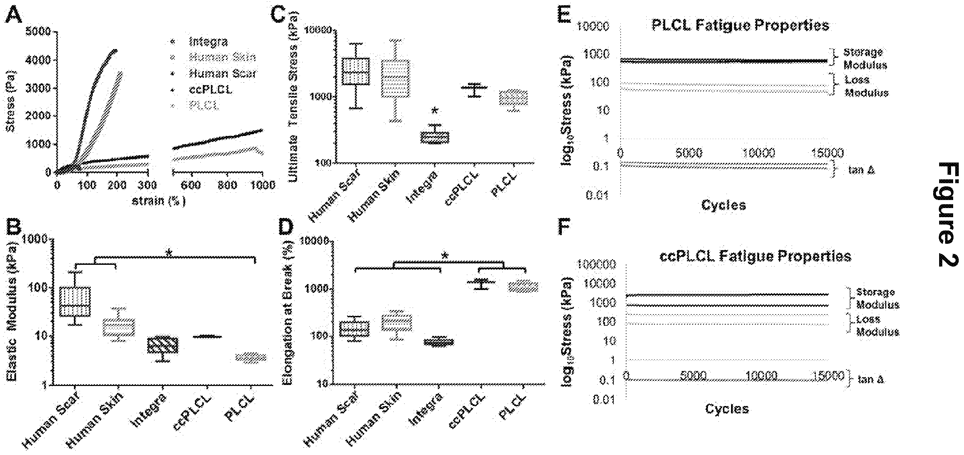

**Figure 2** shows mechanical analysis of PLCL and ccPLCL as compared to Integra, human skin, and human scar. **(A)** Tensile stress- strain curves of human scar (dark gray), human skin (medium gray), Integra (grey-black, thick), ccPLCL (black), and untreated PLCL scaffold (light gray). **(B)** Elastic moduli of PLCL and ccPLCL are less than or equal to that of human skin and scar, suggesting that these materials will not impede patients' range of motion if placed across a joint. **(C)** Unlike Integra, PLCL and ccPLCL scaffolds have similar ultimate tensile stress to human skin and scar. **(D)** Unlike Integra, PLCL and ccPLCL have elongation at break significantly greater than that of human skin and scar, suggesting that these materials will not fail due to tensile forces placed on the skin. **(A-D) (E)** Fatigue properties of PLCL scaffolds. Storage modulus (black), loss modulus (light gray), and tan Δ (dark gray) are all preserved over 15,000 cycles. **(F)** Fatigue properties of ccPLCL scaffolds. Storage modulus (black), loss modulus (light gray), and tan Δ (dark gray) are all preserved over 15,000 cycles. Together, these data show that the clinical standard of care, Integra, is mechanically inappropriate for use beneath skin graft; however, PLCL and ccPLCL scaffolds display appropriate mechanical properties for this application.

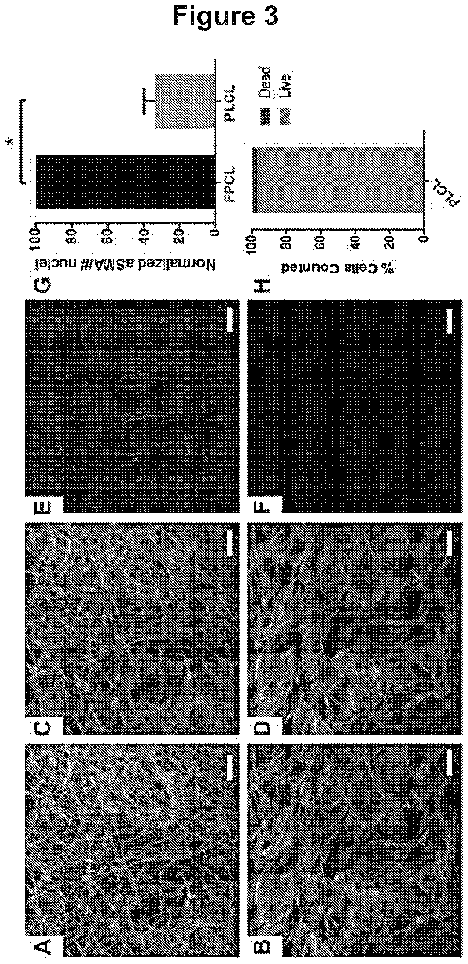

**Figure 3** shows cellular interactions with scaffolds. **(A-F)** Overlay of DAPI (**A** and **B** for FPCLs and PLCL scaffolds, respectivelly), F-actin (**C** and **D** for FPCLs and PLCL scaffolds, respectivelly), and αSMA (**E** and **F** for FPCLs and PLCL scaffolds, respectivelly). **(G)** Significantly less αSMA was present in immunostaining in PLCL scaffolds than in FPCLs. **(H)** Cells remained viable in PLCL scaffolds.

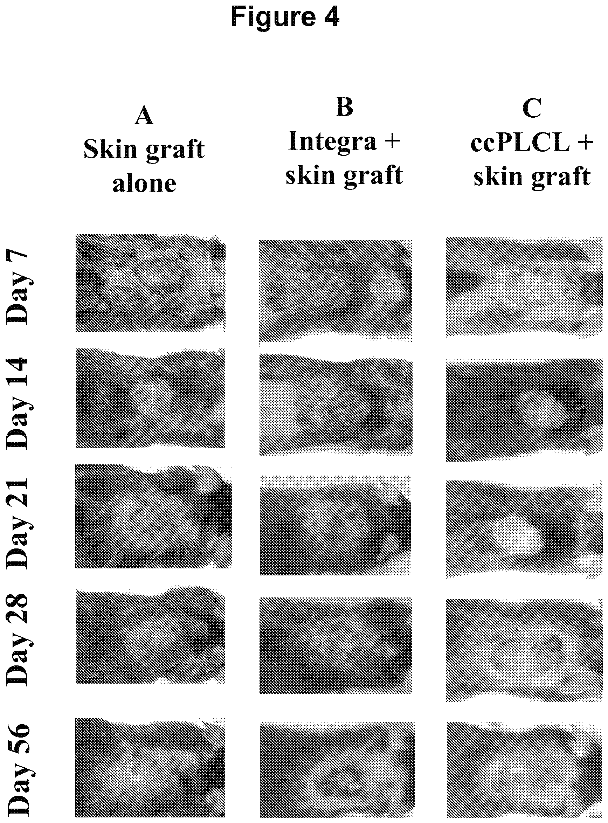

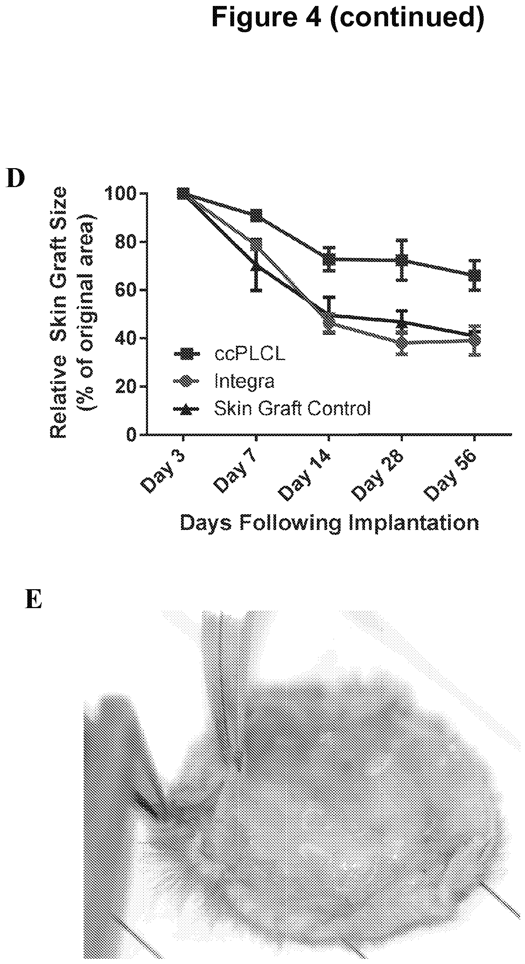

**Figure 4** shows wound contraction and scaffold incorporation out to d56 in vivo. **(A-D)** Gross appearance of wounds treated with **(A)** skin graft alone, **(B)** Integra beneath skin graft, and **(C)** ccPLCL scaffold beneath skin graft, at days 7, 14, 21, 28, and 56 following surgery. **(D)** Wound contraction curves derived from measurements of wounds shown in A-C. **(E)** ccPLCL scaffolds beneath skin graft immediately following excision from wound bed on d30; scaffolds were no longer visibly by naked eye at d56.

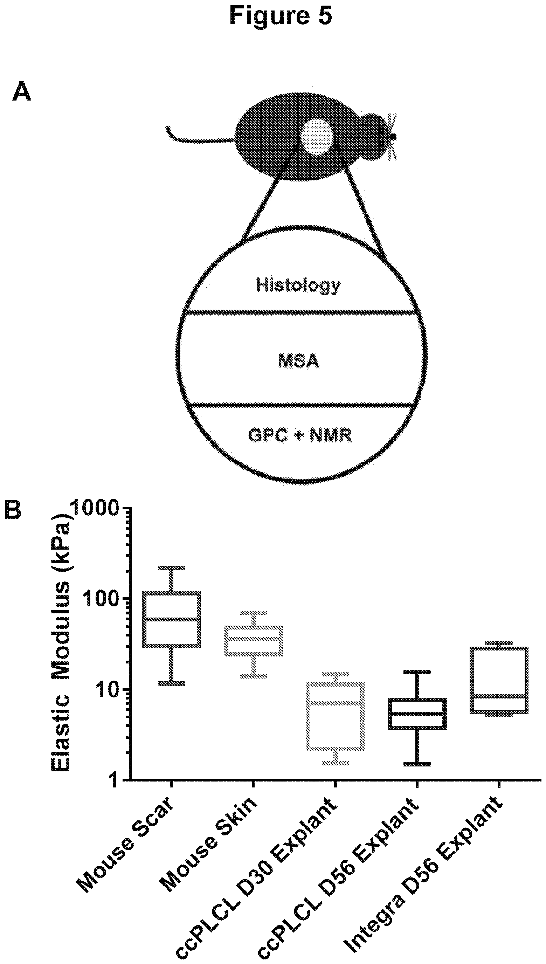

**Figure 5** shows mechanical properties of excised ccPLCL scaffolds on d30 and d56 *in vivo.* **(A)** Wounds were cut into three distinct sections for analysis. **(B)** Tensile testing of explanted ccPLCL and Integra treated wound tissue exhibited elastic moduli significantly lower than skin graft alone treated mice (mouse scar). These data suggest that the ccPLCL scaffold is preventing stiffening associated with HSc scar formation.

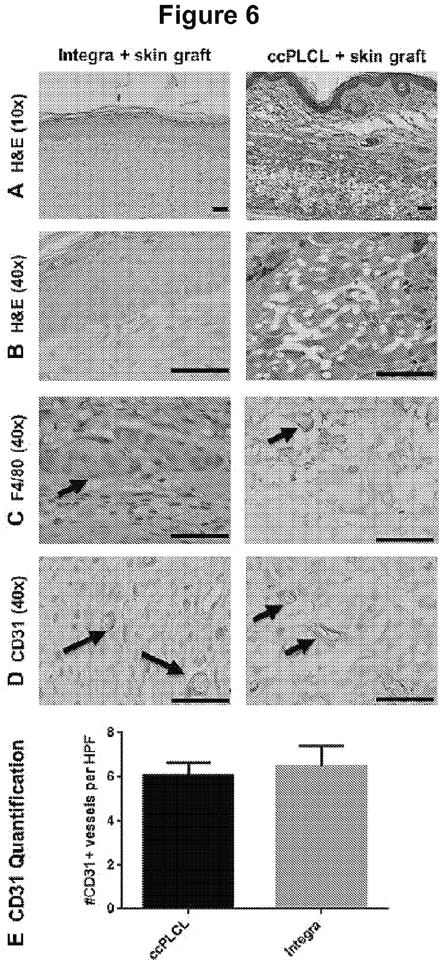

**Figure 6** shows histological analysis of implanted materials at d30. (**A,** left) Low power view of Integra treated wounds shows the presence of skin grafts (epidermis, e) and underlying dermis (d) and dermal scar (s); no evidence of Integra is identified. (**A,** right) ccPLCL treated wounds with overlying skin graft (e), dermis (d) and ccPLCL scaffold (p, outlined); architecture of PLCL scaffold remains intact. (**B,** left) High power view of epidermis and underlying scar in Integra treated wound no evidence of Integra architecture. (**B,** right) Cellular infiltration into PLCL scaffold at d30, ccPLCL (p) architecture can be noted by the existence of white space where the scaffold is present. **(C)** Presence of foreign body giant cells in both Integra treated wound (left) and ccPLCL treated wounds (right) is confirmed by F4/80 pan-macrophage membrane stain (brown). Foreign body giant cells are noted by the presence of black arrows. **(D)** Angiogenesis into wounds is analyzed by staining the endothelial lining of neo-vessels in the wound bed using CD31 slain (brown). Black arrows indicate examples of representative CD31 + vessels. **(E)** Quantitative analysis of CD31 stained sections is graphically represented. Number of CD-31 positive vessels were counted in five 40× high power fields (HPF). There is no significant difference in vascularity in ccPLCL Scaffold compared to Integra-treated wounds at d30 *in vivo.*

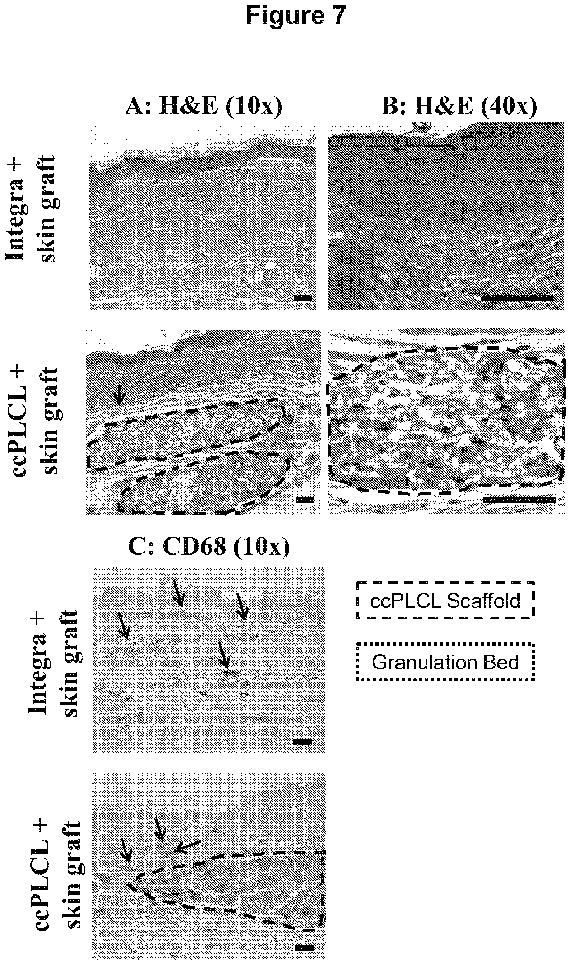

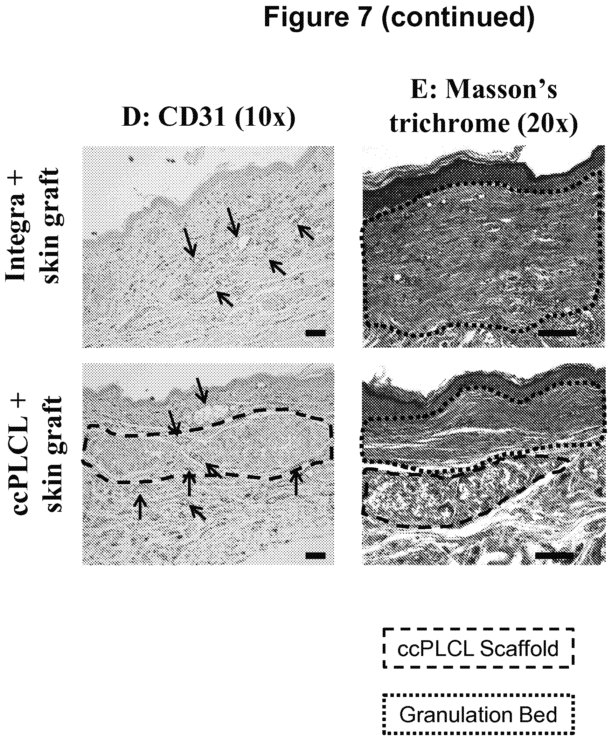

**Figure 7** shows histological analysis of implanted materials at d56. (**A,** left) Low power view of Integra treated wounds shows the presence of skin graft, underlying dermis and dermal scar; no evidence of Integra is identified. (**A,** right) ccPLCL treated wounds with overlying skin graft, dermis, and ccPLCL scaffold (outlined); architecture of PLCL scaffold remains intact. (**B,** top) High power view of epidermis and underlying scar in Integra treated wound no evidence of Integra architecture. (**B,** lower) Cellular infiltration into PLCL scaffold at d56, ccPLCL architecture can be noted by the existence of white space where the scaffold is present (outlined). **(C)** Presence of foreign body giant cells in both Integra treated wound (left) and ccPLCL treated wounds (right) is confirmed by CD68 activated-macrophage membrane stain (brown). Foreign body giant cells are noted by the presence of black arrows. **(D)** Angiogenesis into wounds is analyzed by staining the endothelial lining of neo-vessels in the wound bed using CD31 slain (brown). Black arrows indicate examples of representative CD31 + vessels. (E) Collagen orientation is demonstrated via Masson's Trichrome staining (blue); the presence of a dense granulation bed can be observed in Integra treated wounds (E, upper), while a less thick granulation bed is visible in ccPLCL treated wounds (E, lower). Scale bar = 50µm.

### DETAILED DESCRIPTION OF THE INVENTION

Unless specifically defined, all technical and scientific terms used herein have the same meaning as commonly understood by a skilled artisan in engineering, biochemistry, cellular biology, molecular biology, cosmetics, and the medical sciences (e.g., dermatology, etc.) and, unless specifically defined herein, are not intended to be limiting. All methods and materials similar or equivalent to those described herein can be used in the practice or testing of the present disclosure, with suitable methods and materials being described herein.

### Definitions

Articles "a" and "an" are used herein to refer to one or to more than one (i.e., at least one) of the grammatical object of the article. By way of example, "an element" means at least one element and can include more than one element.

As used herein, the term "comprising" means that other elements can also be present in addition to the defined elements presented. The use of "comprising" indicates inclusion rather than limitation.

As used herein the term "consisting essentially of' refers to those elements required for a given embodiment. The term permits the presence of elements that do not materially affect the basic and novel or functional characteristic(s) of that embodiment of the invention.

The term "consisting of' refers to methods and respective components thereof as described herein, which are exclusive of any element not recited in that description of the embodiment.

The term "severe thermal injury" and "third degree burn" are used interchangeable and refer to those burns that extend through the depth of the skin, where treatment requires removal of all burned (dead) tissue. Such burns are most likely to develop HSc. In certain embodiments, the compositions and methods described herein are used to prevent HSc in third degree burns.

As used herein, the term "hypertrophic scar" refers to an elevated, red contracted scar characterized by excessive deposition of collagen in the wound bed. These scars can be itchy and painful and do not spread outside of the site of original injury. In certain embodiments, the scar may have a contractile (shrunken) component or a proliferative component. The proliferative and contractile components may be observed together in the same scar or independent of one another.

The term "Hypertrophic scar contraction (HSc)" refers to the process of a hypertrophic scar reducing in size. The term "Hypertrophic scar contracture" refers to the shrunken hypertrophic scar. The area of the scar will be significantly less than the area of the initial injury causing tightness in surrounding skin and webbing across joints.

The terms "wound contraction" and "skin graft contraction" are used interchangeably herein.

The term "fibroblast" refers to the most predominant cell type of the skin and connective tissue which is capable of synthesizing the extracellular matrix and collagen. The term "myofibroblast" refers to a cell that is in between a fibroblast and a smooth muscle cell in differentiation. These cells are a more contractile version of the fibroblast and are not typically found in the skin. Fibroblasts turn into myofibroblasts when they encounter mechanical tension (pulling) or wound-related cytokines (chemicals). Myofibroblasts play a critical role in wound healing and HSc by serving to physically contract the wound, secrete new ECM, and remodel the ECM.

As used herein, the term "collagen" refers to a protein synthesized by cells during wound healing and the primary component of the extracellular matrix of skin.

The term "extracellular matrix (ECM)" refers to a collection of proteins and molecules secreted by cells which provide the structural and biochemical support to all tissues within the body. In the skin, the ECM is supported by elastin and glycosaminoglycans, but is primarily made up of collagen.

The term "alpha-smooth muscle actin" or "αSMA" refers to a protein that is characteristically expressed by myofibroblasts and plays a key role in their contractility.

As used herein the term "scaffold" refers to any composition/material that provides structural support for cells in the body of a subject.

The term "polymer" refers to a compound having many repeating units. As used herein, polymers can be synthetic (man-made), or biologic (derived from animals, or recombinantly produced). Synthetic polymers are low cost, have a long shelf life, and possess tunable degradation rates and mechanical properties. However, synthetic polymers do not have cell-specific recognition sites. As a result, certain embodiment of the disclosure provide the biocompatible scaffold that is coated with one or more extracellular matrix proteins. Biologic polymers are high cost, have short shelf-life, possess possibilities of immune-reaction, have weak mechanical properties, and low tunability of degradation rate. However, they have cell-specific recognition sites which allow the body to interact with them readily and easily. The ECM component collagen can be considered a biologic polymer.

As used herein, the term "electrospinning" refers to a low-cost fabrication method used widely in the textile industry to create fabric-like materials with features on the nano-micron size range. In some embodiments, the polymer scaffolds described herein are produced by electrospinning.

Also disclosed are methods of preventing and/or reducing scarring of a wound in a subject.

Thus also disclosed are methods of preventing and / or reducing scarring of a wound in a subject comprising, consisting of, or consisting essentially of implanting a biocompatible electrospun scaffold as described herein in a wound or beneath a skin graft in a wound of the subject. Without being bound to a particular theory, it is believed that implanting the biocompatible electrospun scaffold promotes granulation tissue formation and prevent skin graft contraction.

Yet also disclosed are methods of preventing and/or reducing scar contracture in a wound of a subject comprising, consisting of, or consisting essentially of implanting a biocompatible electrospun scaffold as described herein in the wound, or beneath a skin graft in the wound, of a subject. Without being bound to a particular theory, it is believed that implanting the biocompatible electrospun scaffold minimizes mechanical strain transmission and/or reduce inflammation and ECM alignment thereby preventing and/or reducing scar contracture.

In embodiments, the biocompatible scaffold is implanted subcutaneously.

Any appropriate wound can be repaired according to the methods provided herein. For example, in some embodiments the wound may comprise a chronic wound, such as a venous stasis ulcer or a diabetic foot ulcer. In other embodiments, the wound comprises fibrosis following trauma, thermal injury or radiation damage. In yet other embodiments, the wound comprises a surgical wound, such as those following aesthetic surgery, hernia repair, dura repair, orbital floor repair, breast reconstruction, urological repair, gynecological repair, combinations thereof and the like.

In certain embodiments, the biocompatible matrix is resorbed and/or remodeled by infiltrating components and supportive tissues that are generated or regenerated in accordance with the disclosed methods.

The present disclosure provides a biodegradable scaffold for use in the reduction and/or prevention of scarring in a subject. The biodegradable scaffold preferably comprises superior characteristics of mechanical strength, biodegradation rate, and biocompatibility that will replace or augment Integra™ or analogous skin equivalents. In one embodiment, the biocompatible material comprises poly(l-lactide-co-ε-caprolactone) (PLCL), which comprises a mixture of poly(lactic acid) (PLA) and poly(ε -caprolactone)(PLC). PLCL exhibits elastomeric characteristics because of phase separation of the crystalline PLA and the amorphous PCL segments, creating hard and soft domains that are akin to that observed in elastomeric polyurethanes.

In some embodiments, the PLCL comprises a mixture of PLA and PLC. PLA and PLC are present in a ratio of about 5:95 to about 95:5. In one embodiment, the PLCL comprises a mixture of PLA and PLC, wherein the PLA and PLC are present in a ratio of about 10:90, or about 20:80, or about 30:70, or about 35:65, or about 40:60, or about 45:55, or about 47:53, or about 49:51, or about 50:50, or about 51:49, or about 53:47, or about 55:45, or about 60:40, or about 65:35, or about 70:30, or about 80:20, or about 90:10. In other embodiments, the PLCL comprises a mixture of PLA and PLC, wherein PLA and PLC are present in a ratio of about 10:90 to about 90:10, or about 20:80 to about 80:20, or about 25:75 to about 75:25, or about 30:70 to about 70:30, or about 40:60 to about 60:40, or about 42:58 to about 58:42, or about 45:55 to about 55:45, or about 47:53 to about 53:47, or about 48:52 to about 52:48, or about 49:51 to about 51:49. In some embodiments, the PLCL comprises a 50:50 mixture of PLA and PLC. It will be appreciated that altering the ratios of PLA and PLC will provide the scaffold with different properties, such as elasticity, degradation rate, toughness and the like. Some of these properties may be beneficial depending on the use, and will be readily appreciated by one skilled in the art. Hence, PLCL scaffolds having varying ratios of PLA and PLC are contemplated and intended to be within the scope of the present disclosure.

For example, in some embodiments, the PLCL (50:50) copolymer is synthesized by ring-opening polymerization of l-lactide and e-caprolactone with stannous octoate as the catalyst. In exemplary embodiment, the Mn and Mw as measured by GPC are 186,000 and 305,000, respectively. When compared with PLGA (70:30; Mn=132,000), the macroporous PLCLs were softer but showed an elongation at break that ranged from 200%-500% as the porosity increased from 60 to 90%. Samples having about 60% porosity exhibited a recovery of over 97% at strain applied up to 500%. As the porosity increased to 90%, the samples still showed 100% and 85% recovery at 100% and 200% strain, respectively. Further, when subjected to cyclic strain at 10% amplitude and 1 Hz for 27 days in culture medium (M199) at 37°C, the 60% porous samples maintained their mechanical integrity with only a 10% deformation, whereas the 90% porous samples failed only after two weeks. This range of mechanical characteristics is useful for the methods provided herein as it allows for normal motion of the above-placed skin graft without scaffold rupture or loss of mechanical properties.

In certain embodiments, the biocompatible scaffolds of the disclosure are electrospun. Electrospinning can produce fibers mimicking the topographical features in the ECM of tissues. Topography close to biological scale, in the micron and nanometer range, provides a passive approach without bioactive agents to modulate cell behavior. Further, feature diameter in the range of 4-6 µm has been associated with anti-fibrotic events in coronary tissue. Electrospun fibers also have a high surface area-to-volume ratio that increases cell contact area.

In some embodiments, the biocompatible scaffolds of the disclosure have thickness of about 50 µm to about 5 mm. In other embodiments, the biocompatible scaffolds of the disclosure have thickness of about 50 µm to about 2 mm, or about 50 µm to about 1 mm, or about 50 µm to about 750 µm, or about 50 µm to about 500 µm, or about 50 µm to about 300 µm, or about 50 µm to about 100 µm, or about 100 µm to about 2 mm, or about 100 µm to about 1 mm, or about 100 µm to about 750 µm, or about 100 µm to about 500 µm, or about 100 µm to about 300 µm, or about 0.5 mm to about 5 mm, or about 0.75 mm to about 5 mm, or about 1 mm to about 5 mm, or about 1.5 mm to about 5 mm, or about 2 mm to about 5 mm, or about 3 mm to about 5 mm, or about 0.5 mm to about 3 mm, or about 0.75 mm to about 3 mm, or about 1 mm to about 3 mm, or about 1.5 mm to about 3 mm, or about 2 mm to about 3 mm.

In other embodiments, the biocompatible scaffolds of the disclosure have average pore sizes of about 10 µm to about 300 µm. In some other embodiments, the biocompatible scaffolds of the disclosure have average pore sizes of about 10 µm to about 200 µm, or about 10 µm to about 150 µm, or about 20 µm to about 300 µm, or about 20 µm to about 200 µm, or about 20 µm to about 150 µm, or 30 µm to about 300 µm, or about 30 µm to about 200 µm, or about 30 µm to about 150 µm, or about 30 µm to about 100 µm, or about 50 µm to about 300 µm, or about 50 µm to about 200 µm, or about 50 µm to about 150 µm, or about 50 µm to about 100 µm.

In some embodiments, the scaffold may be coated with extracellular matrix proteins to improve host-scaffold interactions. In some embodiments, this coating is collagen I, collagen III, or hyaluronic acid.

Another aspect of the present disclosure provides that the biocompatible scaffolds may be combined with one or more additional compounds. The one or more compounds may be coated onto the scaffold itself, or administered before, concurrently, and/or after the implanting of the scaffold within the wound. Further, the one or more compounds may be administered at the same time, sequentially, etc. The compounds may include any compound that promotes host/scaffold interactions and/or has anti-scarring and/or pro-wound healing properties. Examples include, but are not limited to, extracellular matrix proteins such as collagen and hyaluronic acid, anti-scarring compounds such as statins and losartan, pro-healing compounds such as peptides, nucleic acids, micro inhibitory RNAs (miRs), antibodies, growth factors and the like. One skilled in the art can readily determine the most optimal route of administration, dosage, and timing of administration of the said one or more additional compounds.

Those of ordinary skill in the art will readily understand that modifications to the biocompatible scaffold structure and/or the use of various compounds, suspensions, solvents, or other materials can customize the rates of agent dispensation. Moreover, dispensation may be controlled as a function of the rate of biocompatible scaffold biodegradation. It is to be further understood that various modifications may be made to the biocompatible scaffold in order to alter the degradation rate of the scaffold structure as a whole, or for only a portion of the scaffold structure. Thus, the compound release may be coordinated with the biodegradation of part or all of the scaffold structure.

### EXAMPLES

### Example 1: Materials and Methods

### Example 1.1: Synthesis of PLCL

PLCL (50% LA, 50% CL) was synthesized as described previsously. Briefly, L-lactide (100 mmol; Purac; Lincolnshire, II., USA) and ε-caprolactone (100 mmol; Sigma; St. Louis, MO, USA) were polymerized at 150 °C for 24 h in the presence of stannous octoate (1 mmol, Sigma) as a catalyst. After being dissolved in chloroform, the polymer was precipitated in methanol, then dried under a vacuum for 72 h and stored in vacuum pack at -20 °C.

### Example 1.2. Fabrication & analysis of electrospun PLCL

PLCL scaffolds were fabricated using continuous single fiber electrospinning to deposit a 3D matrix of fibers on a rotating grounded mandrel using a custom spinning apparatus. PLCL was dissolved in 14% (w/w) dichloromethane overnight. Random fibers were spun at a flow rate of 3 mL/h with a voltage of 8 kV at a distance of 13 cm from the mandrel, which was rotating at ∼70 revolutions per minute. Ambient temperature was 22 ° C with 43% humidity. Following spinning, fibers were removed from the mandrel and residual solvent was removed by air drying for 72 h. Fiber characteristics and scaffold thickness were analyzed using scanning electron microscopy (FEI XL30 SEM-FEG, Hillsboro, OR, USA).

### Example 1.3. Oxygen plasma treatment and collagen coating methods

Samples were placed inside of a plasma asher (Emitech K-1050X, Montigny-le-Bretonneu, France) and treated with reactive oxygen plasma for 45 seconds at 100 W to improve hydrophilicity prior to cell culture and prepare for covalent collagen coating. Following treatment, samples were immediately immersed in sterile water and subsequently sterilized in 70% ethanol for 20 min. Samples were rinsed thoroughly with water following sterilization. Covalent collagen coating was performed by EDC/NHS chemistry as previously described. This well-characterized method is biocompatible, non-cytotoxic, and does not include a linker-arm. Carbiodiamide is not incorporated into the covalent-linkage, allowing the collagen to directly coat scaffold. This method does not modify scaffold morphology and generates a uniform collagen coating covering fibers throughout the depth of the scaffold. Scaffolds in collagen coated PLCL (ccPLCL) groups were covalently coated with bovine type-I collagen (Nutragen, Advanced Biomatrix, San Diego, CA. USA) prior to *in vivo* implantation. Contact angle analysis before oxygen plasma treatment, after oxygen plasma treatment, and after collagen coating treatment was carried out on PLCL films using a goniometer as previously described.

### Example 1.4: Permeability measurement

In order to examine the impact of collagen coating on scaffold permeability, effective hydraulic permeability of PLCL and ccPLCL scaffolds was measured according to ASTM protocol F2952 using a custom-built flowmeter as previously described. In brief, a "scaffold sandwich" was constructed between two silicone gaskets using a 100 µm scaffold and a fine stainless steel mesh. The "sandwich" was placed between mount pieces and a watertight seal was formed by applying light pressure using a threaded screw housing unit. A 50 mL pipette was suspended horizontally 32 cm above the flow chamber and connected. to the specimen mount using tubing. Phosphate buffered saline (PBS) fluid flow was equilibrated for 15 min before timing, and was measured at 1 mL intervals for the first 5 mL, then 5 mL intervals for the next 10 mL in order to ensure consistent flow. The total duration of flow (15 mL) was measured and used to obtain the Darcy coefficient, τ, and average pore diameters as previously described.

### Example 1.5: Static tensile testing of electrospun scaffolds as compared to skin and scar tissues

Static tensile testing was carried out as described in ASTM D3822-07. Human and murine skin and scar tissues were tested as previously described. In brief, scar and uninjured skin samples were gathered from human and murine donors. Uninjured murine tissue samples were collected from the dorsum of 10-12 week-old C57BL/6 mice. Murine scar tissue was taken from contracted d30 skin grafted mice. Tissue from five murine donors was used, with three biological replicates per donor. Human skin samples were donated from discarded human tissue from hospital operating rooms. Uninjured human skin was gathered from breast resection, while scar tissue was taken from excised keloid, radiated forearm. and rejected skin graft. Tissue from three human donors was used with 3-5 biological replicates per donor. All human and murine tissues were kept moist on damp gauze between collection and mechanical testing, and analyzed within 1-5 h of collection. Prior to testing, underlying tissue was removed and samples were cut into uniform strips, PLCL, ccPLCL, Integra, and tissue samples were cut to 5 cm x 5 mm strips using a scalpel and surgical scissors and loaded with a 5 mm gap between clamps. Samples were analyzed on a microstrain analyzer(MSA) (RSA II, TA Instruments, New Castle, DE, USA) at a rate of 0.1 mm/s at room temperature (23 °C) until failure. The initial elastic modulus (within the first 0-200% strain) was analyzed for each sample. The lower elastic modulus was selected for analysis because this strain range best mimics strains that are experienced in the body. Ultimate tensile strength and elongation at break were taken from the data set of each sample.

### Example 1.6: Fatigue testing of PLCL and ccPLCL scaffolds

Fatigue properties of scaffolds were measured to determine whether the samples would be able to withstand continuous cyclic loading, such as that encountered across joints in the body. PLCL and ccPLCL samples were loaded on the MSA and immersed in 37 °C PBS using a heated sample cup. Samples were allowed to equilibrate for 1 h, then extended to 10% strain, and oscillated over 10% strain at 1 Hz for 24 h according to ASTM protocol D3479/D3479M-12.

### Example 1.7: Cell culture

Adult normal human dermal fibroblasts (NHDFs) (Lonza, Basel, Switzerland) were cultured in high glucose Dulbecco's Modified Eagle's Medium (GIBCO 11960-044) (Invitrogen, Grand Island, NY, USA) supplemented with 10% Premium Select fetal bovine serum (FBS) (Atlanta Biologicals, Lawrenceville, GA, USA), 25 µg/mL gentamicin (Invitrogen), and 1 x GlutaMAX, non-essential amino acids, sodium pyruvate, and P-mercaptoethanol (Invitrogen), at 37 °C and 5% CO₂. NHDFs were passaged a maximum of six times prior to experimentation.

### Example 1.8: Contraction studies

Media was prepared fresh on d1 (10% FBS + 5 ng/mL TGFβ), fresh media was added at d2.5, and the trial was stopped at d5 for analysis. Following sterilization, PLCL and ccPLCL scaffolds (8 mm round, 60 µm thick) were washed with PBS and left in 10% FBS media for 24 h prior to cell culture studies. Prior to seeding. scaffolds were rinsed thoroughly with PBS, placed in a 48 well suspension plate, and seeded with 200,000 cells in 500 µL media. Cells were allowed to attach for 1 h before addition of 500 µL media.

The *in vitro* three dimensional fibroblast populated collagen lattice (FPCL) assay was originally developed in 1979. The FPCL was the progenitor assay that led to the development of today's clinical standard of care treatment. The FPCL remains the gold-standard for understanding how fibroblast contractility causes wound contraction. The FPCL assay is the closest available in vitro model of scar contraction because scars and FPCLs both (1) are composed of collagen type I, (2) contain fibroblasts and myofibroblasts, and (3) are contracted by cellular forces. FPCL contraction is expedited by substances promote HSc contraction, and substances that inhibit FPCL contraction inhibit HSc contraction. Fibroblast behavior observed in FPCLs closely mimics *in vivo* observations in healing scars including fibroblast alignment, myofibroblast formation, and collagen production. FPCLs can be used to model initial wound bed contraction (floating matrices) or granulation bed contraction (fixed matrices). Floating FPCLs are mechanically released from the sides of the dishes one hour after gel polymerization. Cells rapidly remodel and contract the collagen similar to the process of initial wound bed contraction. In contrast, attached matrices allow isometric tension to build up as the contractile forces exerted by cells encounter mechanical resistance, which is thought to mimic granulation tissue contraction.

FPCLs were constructed as previously described. In brief. FPCLs were prepared in triplicate by combining the following materials in the corresponding order. 50 µL of 5×PBS (pH 8.5, 23 °C) was combined with 200 µL bovine type I collagen (6 mg/mL, pH 5, 23 °C) (Nutragen, Advanced Biomatrix. San Diego, CA, USA), and 750 µL cell suspension (590,000 cells/mL). FPCLs were cast in small, prewarmed, triplicate 35 mm x 10 mm tissue culture polystyrene dishes (250 µL/dish). Dishes were placed in a 37 °C cell culture incubator for 1 h to allow FPCLs to solidify before slowly adding 2.5 mL media. FPCLs were allowed to remain attached to the cell culture dish throughout the study.

### Example 1.9: Viability analysis

Cellular viability analysis was performed using LifeTechnologies Live/Dead Viability/Cytotoxicity Kit for Mammalian Cells (Invitrogen). Live/dead staining on d5 NHDF-seeded PLCL and ccPLCL scaffolds was conducted according to manufacturer instructions. D5 scaffolds were mounted and imaged on an inverted fluorescent microscope (Eclipse TE2000-U, Nikon, Tokyo, japan) within 3 h. Images were analyzed by counting labeled cells using Image J software (NIH).

### Example 1.10: Immunocytochemistry

Cells were fixed with 4% paraformaldehyde in PBS for 15 min at room temperature, permeabilized, and stained in a blocking solution containing the antibodies, 0.03 g/mL bovine serum albumin (BSA, Sigma), 10% goat serum (Sigma), and 0.3% Triton X-100 (Sigma) in PBS. Samples were blocked for 3 h prior to staining. Primary stain for αSMA (1:100, Abcam, Cambridge, MA, USA) was conducted for 18 hat 4 °C, the samples were then washed before incubation with Alexa Fluor 594 anti-mouse secondary antibody (1:200, Invitrogen) for 4 h at room temperature. Cell nuclei were stained with 4,6-diamidino-2-phenylindole (DAPI) (1:5000, Invitrogen) and the actin cytoskeleton was stained with phalloidin 488 (1:200, Invitrogen). The samples were then mounted in Fluoro-gel (Electron Microscopy Sciences, Hatfield, PA, USA) for fluorescent imaging.

Images for analysis of αSMA presence in immunocytochemistry were acquired using an inverted confocal microscope (Zeiss LSM 510, Oberkochen, Germany) with 10× magnification. Three images from each of three replicates were collected for each condition, resulting in a minimum of 2000 cells for each condition for analysis using Image J software. Nuclei quantification and area of αSMA positive stress fibers were analyzed using a size exclusion to disregard non-cell debris and αSMA localized to the cytosol or focal adhesions. Quantification of αSMA positive stress fibers was achieved by dividing the area of αSMA by the number of nuclei present in the image and normalizing to staining in FPCLs as 100.

### Example 1.11: Statistical Analysis

Unless otherwise stated, the following applies to all experimental results. Gaussian data have been presented as mean ± standard error of the mean. Two-way ANOVA followed by t-test was carried out to discern differences between groups, with significance at p < 0.05. In the case of non-Gaussian results, data were presented in box and whisker format. Significance was analyzed via two-way ANOVA followed by Mann-Whitney test. Mechanical and permeability analysis experiments were carried out with n ≥ 5, in vitro experiments were carried out in triplicate at least three separate times, and *in vivo* experiments were conducted with n ≥ 5 per treatment group.

### Example 2: Surgical Methods

Female C57BL/6 mice, 10-12 weeks-old, weighing 18-23 g (Jackson Laboratories, Bar Harbor, ME), were used as wild type mice throughout the study. Mice were housed individually for the first three weeks, and in groups of four for the final week of study. Female mice were selected to reduce the risk of aggression-related injury when transferred to group housing; only one sex was used in order to reduce secondary variables related to hormonal differences between male and female mice. Surgical methods were performed as described previously; in all instances, skin graft refers to the use of a split thickness skin graft fashioned from the skin tissue of two donor mouse ears. In brief, a third degree burn was created on the dorsum of the donor mouse, the bum was left for 3d, and a 14 mm diameter circle over the burn site was excised for recipient skin grafting. Treatment groups included: (1) skin graft without placement of scaffold material, (2) skin graft over Integra, (3) skin graft over PLCL, (4) skin graft over ccPLCL. Scaffolds 110 µm in thickness ± collagen coating were cut into circles using a 1.2 cm diameter arch steel hole punch prior to sterilization. Integra was cut using a 1.2 cm punch immediately before surgery and removed from the silicone backing. Integra, sterile PLCL, or sterile ccPLCL scaffolds were laid into the wound bed and sutured just beneath the skin edges with four mattress stitches of 6-0 silk suture. Donor ear skins were laid over the excised burn wound after the implantation of the relevant scaffold to fashion a skin graft. The murine skin grafts were then secured with a padded bolster. The bolster was removed on postoperative d3.

### Example 3: Analysis of electrospun scaffold mechanical properties and degradation on d30 in vivo

The mice were euthanized and tissue was collected on postoperative d30 in all mice. The collected tissues from d30 and d56 mice treated with ccPLCL, Integra, or skin graft alone (scar tissue) were cut into three pieces (Fig. 5A). The two peripheral tissue specimens were preserved in 10% formalin and embedded in paraffin wax for histological analyses. The center piece was immediately taken to MSA for static tensile testing. Tissue ex plants were kept moist between collection and mechanical testing, and analyzed within 2 h of collection.

PLCL was extracted from d30 excised tissue samples by incubation with chloroform overnight and tested using nuclear magnetic resonance (NMR) and gas permeation chromatography. The component composition of PLCL was analyzed using a 400 MHz ¹H NMR. Spectra were obtained using 1% (w/v) solutions in CDCl₃ and the compositions calculated from these relative intensities. Molecular weights were determined using a gel permeation chromatography. Chloroform was used as the mobile phase at a flow rate of 1 mL/min. Calibration was performed using polystyrene standards to determine number-average and weight-average molecular weights (Mₙ and M_{w}).

### Example 4: Immunohistochemistry

Staining was carried out according to routine procedures. Primary antibodies used included: F4/80 (1:1500, eBioscience, San Diego, CA, USA) and anti-CD31 (1:50, Abeam). Secondary antibodies included: biotinylated rabbit anti-rat (1:200, Vector Laboratories, Burlingame, CA, USA), biotinylated goat anti-rabbit (1:50, Vector Laboratories), avidin-biotin complex reaction (Vector Laboratories), DAB substrate solution (Biocare Medical, Concord, CA, USA). Stained slides were visualized by use of a Nikon eclipse E600 microscope and images were captured with a Nikon DXM 1200 digital camera under the same settings. Quantification of CD31 images was performed using five HPF images per section across a minimum of five mice. Only positively stained vessels inside the perimeter of the scaffolds were quantified. Fiber diameter of ccPLCL scaffolds at d30 *in vivo* was calculated using H&E images, one HPF per mouse and a minimum of ten measurements were used.

### Example 5: Scaffold characteristics

Scaffolds were spun to thickness of 60 ± 10 µm for in vitro studies and 100 ± 11 µm for *in vivo* studies; fiber diameter was 5.6 ± 0.70 µm for all samples (Fig. 1A,B). We chose to use a fiber diameter in this range because it has been associated with antifibrotic events in coronary tissue. Covalent collagen coating uniformly covered fibers throughout the depth of the scaffolds (Fig. 1C) and did not significantly modify fiber diameter (6.5 ± 0.66 µm) or scaffold morphology (Fig. 1 D). Contact angle analysis before oxygen plasma treatment, after oxygen plasma treatment, and after collagen coating treatment on PLCL films showed progressive hydrophilicity of 72 ± 6°, 60 ± 1°. and 44 ± 8°, respectively (Fig. 1 E).

### Example 6: Permeability analysis

PLCL scaffolds exhibited a mean Darcy permeability constant, τ, of 20.0 ± 1.2 µm², compared to 19.7 ± 1.6 µm² for ccPLCL scaffolds with no statistically significant difference (Fig. IF). The corresponding average pore sizes were 4.54 ± 0.13 µm and 4.50 ± 0.18 µm, respectively. Similarly, measured pore size using SEM images found an insignificant difference in average pore size of PLCL scaffolds (40.8 ± 2.8 µm) and ccPLCL scaffolds (36.3 ± 2.3 µm), further confirming that the collagen coating did not affect scaffold pore size. The discrepancy between derived average pore size and SEM measured pore size has been previously described when calculating pore size of electrospun scaffolds using this technique. Sell et al.(J Biomed Mater Res Part A 2008, 85A(1):115-26) postulated that these difference may stem from the presence of "faux pores" and blind pouches within the scaffolds which are not visible via SEM measurement but affect the effective pore size gathered from flowmeter measurement.

### Example 7: Mechanical properties

The mechanical properties of PLCL and ccPLCL scaffolds were analyzed using MSA and compared to those of human skin, human scar, and Integra (Fig. 2A). The elastic modulus of uncoated PLCL (3.6 ± 0.22 kPa) was significantly lower than that of Integra (6.5 ± 0.91 kPa), human skin (17 ± 1.6 kPa), and scar (55 ± 14 kPa). Elastic modulus of ccPLCL (83 ± 0.67 kPa) was not significantly different from that of Integra, suggesting that the combined process of oxygen plasma treatment and collagen coating increased the PLCL scaffold stiffness (Fig. 2B). Oxygen plasma treatment is known to generate a higher crosslinking density within the first few thousand angstroms of polymer surface, which results in a local increase in hardness and likely attributes to the modest increase in elastic modulus of ccPLCL.

The ultimate tensile stress (UTS) for both PLCL(0.97 ± 0.10 GPa) and ccPLCL scaffolds (1.3 ± 0.17 GPa) was significantly greater than that of Integra (0.26 ± 0.020 GPa). While PLCL showed lower UTS than that of human skin (2.6 ± 0.040 GPa), or human scar (2.7 ± 0.50 GPa), ccPLCL did not (Fig. 2C). In contrast, the elongation at break (EB) for PLCL (1100 ± 93 kPa) and ccPLCL (1300 ± 170 kPa) scaffolds was significantly higher than the values obtained for Integra (75 ± 4.4 kPa), human skin (200 ± 16 kPa), and human scar (140 ± 16 kPa) (Fig. 2D). The storage modulus, loss modulus, and tanΔ of PLCL and ccPLCL scaffold subjected 10% strain at 1 Hz showed negligible deterioration over 24 h, or 15,000 cycles (Fig. 2E). This fatigue testing condition was chosen to emulate joint range of motion at the pace of walking. Taken together, the mechanical properties of PLCL and ccPLCL scaffolds relative to human skin and scar - lower modulus, comparable UTS, and higher elongation at break - suggest that they would be suitable materials for placement beneath a skin graft.

### Example 8: In vitro analysis of HSc-related outcomes

Immunostaining was used to study cell morphology and as a semi-quantitative method to analyze expression of αSMA in stress fibers. Cells seeded in FPCLs displayed distinct actin stress fibers with heavy αSMA incorporation (Fig. 3A-C). Cells seeded in PLCL scaffolds displayed larger size, with more diffuse actin stress fibers and little αSMA incorporation (Fig. 3D-F). At d5 following seeding, significantly fewer cells had converted to myofibroblast phenotype in PLCL scaffolds (34 ± 6.2%), as compared to those seeded in FPCLs (normalized to 100%) (Fig. 3G). Cells remained viable in PLCL and ccPLCL scaffolds (Fig. 3H) with 97 ± 13% viable cells at d5 in PLCL scaffolds.

### Example 9: In vivo application of PLCL and ccPLCL scaffolds

All data presented below are described in terms of percentage of original wound size, where 100% is the wound size at d3 (after removal of the post-operative bolster), and a fully contracted wound would be described as 0% of its original size. All samples were applied beneath the skin graft. Murine wounds treated with skin grafts alone contracted to 47 ± 2.0 % at d30 (Fig. 4A), while wounds treated with Integra contracted to 28 ± 1.8% (Fig. 4B). Wounds treated with uncoated PLCL scaffolds remained at 68 ± 11% at day 21. However, partial scaffold extrusion and skin graft death began at d21 and continued until the end of the study, resulting in wound contraction down to 22 ± 11% by d30 (Fig. 4C). In contrast, wounds treated with ccPLCL scaffolds showed significantly decreased contraction, down to 95 ± 5.8% at d30 (Fig. 4D). Fig. 4E shows the rate of wound contraction over the test period. Upon extraction of the tissue from the mice on d30, ccPLCL scaffolds were observed to integrate with host tissue beneath the skin grafts, indicating that it out-performed all other groups in delaying HSc contraction (Fig. 4F).

### Example 10: Physicochemical properties of scaffolds at d30 after implantation

Tensile testing of explanted ccPLCL from d30 mouse studies displayed a similar elastic modulus to scaffolds prior to implantation (Fig. 5B). d30 ccPLCL explants exhibited an elastic modulus (5.7 ± 2 kPa) significantly lower than tissue alone in both mouse skin (36 ± 3.3 kPa) and scar (80 ± 17 kPa) samples, although not significantly different from that prior to implantation (9.7 ± 0.20 kPa). The molecular weight of the scaffolds decreased by 49% from Mₙ = 151 kDa to Mₙ = 73.2 ± 6.5 kDa over the implanted period of 30 days, and to Mₙ = 17 at 56 days. NMR analysis shows that the lactide (LA) moiety was noticeably more rapidly decreased than the caprolactone (CL) units at 30 days and 56 days. The molar ratio was as expected since PLA is known to degrade more rapidly than PCL. The mole fraction of LA decreased from 50% to 27% in 56 days, while that of CL increased from 50% to 73%. Table 1 displays results of NMR and GPC analysis.

**Table 1. Analysis of explanted scaffold material. Mₙ, M_{w}, and PDI were gathered using GPC, LA:CL ratio was found via NMR.**

| | Mn (kDa) | Mn (%) | Mw (kDa) | Mw (%) | PDI | LA:CL |
|---|---|---|---|---|---|---|
| Initial | 151 | 100.0 | 266 | 100.0 | 1.8 | 50:50 |
| d30 Explant | 73 | 48.6 | 135 | 50.7 | 2.0 | 44:56 |
| D56 Explant | 17 | 11.2 | 33 | 12 | 2.0 | 27:73 |

### Example 11: ccPLCL scaffolds incorporate into host tissue and maintain architecture in vivo

Fig. 6A-D shows representative images of histological staining Integra and ccPLCL scaffold treated wounds on d30. Fiber diameter of ccPLCL scaffolds at d30 *in vivo* (5.5 ± 0.33 µm) was not significantly different from initial fiber diameter prior to implantation. Throughout all samples, H&E stained Integra and ccPLCL scaffolds on d30 exhibited acute inflammation (Fig. 6A,B). ECM alignment appeared more prevalent in Integra treated samples than in ccPLCL treated samples (Fig. 6A,B, data not quantified). Negligible fibrous capsule formation was present in ccPLCL treated mice; no fibrous capsule was visible in Integra treated mice as the implant was no longer discernable within the wound bed. CD31 staining demonstrated vessel ingrowth into ccPLCL scaffolds and Integra. Multinuclear giant cells and neutrophils were visible at the boundary of the ccPLCL scaffolds with the tissue (Fig. 6B). F4/80 staining confirmed the presence of macrophage within the giant cells in wounds treated with Integra and ccPLCL (Fig. 6C). Cells penetrating into the ccPLCL scaffold and Integra granulation tissue included histocytes and foreign body giant cells. Quantification of CD31 outlined vessels showed that angiogenesis into ccPLCL (6.1 ± 0.54 vessels/HPF) and Integra (6.5 ± 0.90 vessels/HPF) was not significantly different; vessels were observed spanning both scaffold materials as well as passing through the material (Fig. 6D).

### Examples 1-11: Results

A synthetic, biodegradable, elastomeric, electrospun scaffold was employed to evaluate the effect of scaffold longevity on the development of HSc contraction *in vivo.* In developing a scaffold for use in HSc prevention, the properties of healthy skin were focus of design parameters (e.g., the mechanical properties of human skin, including its tensile and viscoelastic characteristics.) To improve cell-scaffold interactions, bovine collagen was covalently attached to the scaffold prior to *in vivo* implantation. To ensure adequate time for HSc stabilization in skin-grafted murine wounds, all treatment groups were followed out for 30 days following implantation. While wound healing studies are often conducted over 14 days, this extended time line allowed for an early determination of the effects of scaffold degradation on scar contraction. The biodegradable elastomer of the disclosure demonstrates appropriate mechanical properties for implantation beneath skin grafts and is capable of repetitively undergoing physiologically relevant strain and relaxation without entering plastic deformation. Most importantly, this synthetic elastomer possesses a degradation rate on the scale of six months *in vivo,* allowing it to maintain its architecture throughout the remodeling phase of repair.

While traditional BSEs are attractive, their degradation rates and mechanical properties are difficult to design in practice. The mechanical properties of human skin are critical to consider during the design of a scaffold for HSc prevention. Scaffolds with tensile elastic moduli greater than human skin may inhibit joint motion, similar to how stiffened scar inhibits motion. Therefore, BSEs should possess an elastic modulus less than or equal to that of human skin. Further, possessing elongation at break and ultimate tensile stress less than that of human skin may cause a scaffold to rupture beneath the skin prior to completion of healing. Along with its tensile characteristics, skin is viscoelastic in nature, allowing it to stretch and relax across joints repetitively. Burns that occur across joints, especially those of the upper body, are the most common location for HSc to occur. In fact, joint motion during healing is likely a driving factor of HSc. Thus, it is important that BSEs placed in the wound bed are designed with the appropriate elastomeric properties to withstand repeated expansion and relaxation cycles across joints.

In designing a synthetic scaffold for permanent placement in the wound bed, it is critical that cells remain viable in the scaffold and that the scaffold does not exacerbate scarring. Cells seeded in ccPLCL scaffolds remain viable in vitro confirming that electrospun scaffolds provide a suitable cellular micro-environment. Once seeded in the FPCL, fibroblasts are able to contract the collagen fibrils in the gel similarly to how they would contract scar granulation tissue. The fraction of cells which converted into myofibroblasts was analyzed by immunocytochemical staining for αSMA, which is a marker for myofibroblast formation. Using this semi-quantitative method, significantly more αSMA was found in the FPCL than was present in PLCL scaffolds. These data suggest that electrospun PLCL scaffolds mitigate cellular processes associated with HSc.

While uncoated PLCL scaffolds were rejected between d21-d28, ccPLCL scaffolds integrated into the wound tissue and prevented HSc contraction. Without being bound to a particular theory, it is believed that this is likely because synthetic polymer implants do not offer integrin binding sites and often require surface treatment prior to implantation. PLCL scaffolds are hydrophobic in nature, encouraging the likelihood for random protein adsorption and eventual extrusion. The extrusion process is caused by spontaneous and uncontrollable adsorption of proteins from the blood, lymph, and wound exudate to hydrophobic implant surfaces. Implantation of synthetic polymer structures into open wounds without integrin binding sites can lead to foreign body encapsulation and extrusion. To decrease the likelihood of random protein adsorption and encourage cell-scaffold interactions through integrin binding sites, collagen was covalently attached to the surface of PLCL scaffolds. There is concern that protein coating of electrospun polymers may restrict effective aqueous fluid flow, thus disrupting transport of fluids and nutrients through scaffolds *in vivo.* However, despite the presence of a protein coating, a negligible change in effective permeability was found between ccPLCL and PLCL scaffolds. Following *in vivo* implantation, ccPLCL scaffolds integrated into the tissue beneath the skin graft. The beneficial impact of the collagen coating could be explained by favorable changes in scaffold hydrophilicity or the introduction of cell-binding motifs. These data are in agreement with the literature on the importance of introducing integrin binding sites to the surfaces of intra-dermal, hydrophobic, synthetic polymer implants.

Upon removal of wound tissue from the mice, ccPLCL scaffolds could be discerned beneath skin grafts. Tensile testing of explanted ccPLCL from d30 mouse studies displayed a similar elastic modulus to scaffolds prior to implantation (Fig. 5B). d30 ccPLCL explants also exhibited an elastic modulus significantly lower than tissue alone in both mouse skin and scar samples. These data suggest that the scaffold is preventing stiffening associated with HSc scar formation. Scar formation is also associated with alignment of the ECM, as is seen in d30 Integra treated samples via H&E staining. Conversely, cells in ccPLCL scaffolds display random nuclear orientation and ECM alignment at d30 via H&E staining (Fig. 6B). The presence of a foreign body reaction, characterized by neutrophil and macrophage infiltration, can be seen in both treatment groups. The acute inflammation present in these sections is confirmed by F4/80 staining and is commonly observed following intradermal implantation of a foreign body. Vessel infiltration is not significantly different between Integra and ccPLCL scaffolds, suggesting that both materials are integrating well with the host tissue and allowing lymphogenesis and/or angiogenesis to occur. Overall, the histological data suggest the presence of an acute inflammatory reaction in ccPLCL treated mice along with vessel infiltration into the scaffold and maintenance of randomly oriented ECM. These outcomes are all necessary to maintain skin graft health and prevent the formation and subsequent contraction of HSc.

ccPLCL scaffolds significantly prevented HSc contraction *in vivo* as compared to Integra or placement of a skin graft alone. Integra treated mice maintained 75% of the original wound area until day eight, after which scars rapidly contracted (Fig. 4E). The contraction seen after day eight could be associated with partial necrosis of skin grafts on Integra-treated wounds due to the immediate placement of skin grafts over Integra. While collagen-based scaffolds can be successful following immediate skin graft placement, Integra requires a two-step implantation procedure. During clinical use, Integra is placed and allowed to integrate into the wound bed for approximately two weeks prior to placement of the skin graft. This delay allows a period for vascular infiltration of the Integra matrix; however, this method increases risk of infection and requires a second operation. In general, collagen scaffolds, such as Integra, are associated with wound contraction and scarring. Collagen is degraded and remodeled by collagenases and other proteases within the wound bed with a half-life on the order of days to weeks depending on the crosslinking method. It is conceivable that after day eight of implantation, Integra begins to lose its architecture and mechanical properties, allowing rapid scar contraction. A similar rapid contraction after day eight has been shown in the literature following treatment of full thickness dermal wounds with electrospun collagen scaffolds in a guinea pig model. Together, these data suggest that rapid scar contraction after day eight is related to the longevity of collagen scaffolds in the wound bed, rather than the fabrication technique. Wounds also rapidly contracted following extrusion of uncoated PLCL scaffolds, which began after d21 *in vivo* (Fig. 4E). Rapid wound contraction is commonly seen following removal of splinting materials from murine wounds, suggesting that Integra and non-incorporated scaffolds may act as temporary splinting materials to keep the wound open until they either extrude or lose their architecture via degradation.

HSc contraction develops progressively over the course of several weeks in mice and multiple months in humans; therefore, it is feasible that a loss of scaffold architecture prior to the completion of the remodeling phase of repair could allow for delayed cellular alignment and the formation of a mechanically-coordinated cellular syncytium, thus increasing the likelihood of HSc contraction. The structural support provided by collagen-based scaffolds, such as Integra. has been suggested to rely upon the pore architecture of the scaffold, rather than the elastic modulus of the material. The present data support the hypothesis that elastic modulus of the implant does not dictate its ability to resist wound contraction; the elastic modulus of ccPLCL electrospun scaffolds is within the same order of magnitude as Integra when tested in the early strain region. However, the effects on wound contraction between Integra and ccPLCL scaffolds are drastically different at d30 *in vivo.* At d30 following implantation, the architecture of ccPLCL scaffolds remains clearly defined in the wound bed histology, whereas the d30 the architecture of Integra is not visible via histological staining. Lyophilized collagen-GAG based scaffolds, similar to Integra, degrade over the order of several days to several weeks. This short degradation time can be exacerbated by patient-to-patient variability in the wound healing process, leading to the possibility of premature degradation prior to proper healing. Indeed, rapid degradation and resorption of a BSE prior to the completion of the remodeling phase of repair leads to increased likelihood of scar formation. We hypothesize that the critical difference leading to the success of ccPLCL scaffolds in preventing HSc contraction is the residence time of the scaffold architecture in the wound bed. The PLCL material employed in this study will retain its structure in the wound bed throughout the remodeling phase of repair, having only lost 51% of its number-average molecular weight at 30 days *in vivo.*

## Claims

1. A collagen I coated biocompatible electrospun scaffold for use in a method of preventing and/or reducing scarring of a wound in a subject, wherein the method comprises implanting the collagen I coated biocompatible electrospun scaffold in the wound of the subject to promote granulation tissue formation and to prevent wound and scar contraction.

2. A collagen I coated biocompatible electrospun scaffold for use in a method of preventing and/or reducing scar contracture in a wound in a subject, wherein the method comprises implanting the collagen I coated biocompatible electrospun scaffold in the wound of the subject to minimize mechanical strain transmission and/or reduce ECM alignment and inflammation thereby preventing and/or reducing scar contracture.

3. The collagen I coated biocompatible electrospun scaffold for use of claim 1 or 2, wherein the scaffold comprises poly(l-lactide-co-ε-caprolactone) (PLCL).

4. The collagen I coated biocompatible electrospun scaffold for use according to any one of claims 1-3, wherein the collagen I coated biocompatible scaffold is implanted in an open wound or beneath an applied skin graft.

5. The collagen I coated biocompatible electrospun scaffold for use according to any one of claims 1-4, wherein:
- the wound comprises a chronic wound;
- the wound comprises a venous stasis ulcer or a diabetic foot ulcer;
- the wound comprises fibrosis following trauma, thermal injury, or radiation burn; or
- the wound comprises a surgical wound.

6. The collagen I coated biocompatible electrospun scaffold for use according to claim 5, wherein the surgical wound comprises a wound resulting from the group consisting of aesthetic surgery, hernia repair, dura repair, orbital floor repair, breast reconstruction, urological repair, gynecological repair, and combinations thereof.

7. The collagen I coated biocompatible electrospun scaffold for use according to any one of claims 1-6, wherein the scaffold is combined with one or more additional compounds selected from the group consisting of an anti-scarring compound, a pro-healing compound, and an anti-inflammatory compound.

8. The collagen I coated biocompatible electrospun scaffold for use according to claim 7, wherein the one or more additional compounds are selected from the group consisting of a statin, losartan, micro inhibitory RNAs (miRs), peptides, and antibodies.

9. The collagen I coated biocompatible electrospun scaffold for use according to any one of claims 1-8, wherein the scaffold comprises a 50:50 mixture of poly(lactic acid) (PLA) and poly(ε-caprolactone) (PCL).

10. The collagen I coated biocompatible electrospun scaffold for use according to any one of claims 1-9, wherein the scaffold is electrospun to thickness of about 50 µm to about 5 mm.

11. The collagen I coated biocompatible electrospun scaffold for use according to any one of claims 1-10, wherein the scaffold is electrospun to average pore sizes of about 10 µm to about 300 µm.

## Patentansprüche

1. Mit Kollagen I beschichtetes biokompatibles elektrogesponnenes Gerüst zur Verwendung in einem Verfahren zur Verhinderung und/oder Reduzierung von Narbenbildung einer Wunde in einem Individuum, wobei das Verfahren die Implantation des mit Kollagen I beschichteten biokompatiblen elektrogesponnenen Gerüsts in der Wunde des Individuums zur Förderung der Bildung von Granulationsgewebe und zur Verhinderung von Wunden- und Narbenkontraktion umfasst.

2. Mit Kollagen I beschichtetes biokompatibles elektrogesponnenes Gerüst zur Verwendung in einem Verfahren zur Verhinderung und/oder Reduzierung von Narbenkontraktur in einer Wunde in einem Individuum, wobei das Verfahren die Implantation des mit Kollagen I beschichteten biokompatiblen elektrogesponnenen Gerüsts in der Wunde des Individuums zur Minimierung mechanischer Spannungsübertragung und/oder Reduzierung von EZM-Ausrichtung und Entzündung umfasst, wodurch Narbenkontraktur verhindert und/oder reduziert wird.

3. Mit Kollagen I beschichtetes biokompatibles elektrogesponnenes Gerüst zur Verwendung nach Anspruch 1 oder Anspruch 2, wobei das Gerüst Poly(1-lactid-co-ε-caprolacton) (PLCL) umfasst.

4. Mit Kollagen I beschichtetes biokompatibles elektrogesponnenes Gerüst zur Verwendung nach einem der Ansprüche 1-3, wobei das mit Kollagen I beschichtete biokompatible Gerüst in einer offenen Wunde oder unter einem aufgelegten Hauttransplantat implantiert wird.

5. Mit Kollagen I beschichtetes biokompatibles elektrogesponnenes Gerüst zur Verwendung nach einem der Ansprüche 1-4, wobei:
- die Wunde eine chronische Wunde umfasst;
- die Wunde ein venöses Stauungsgeschwür oder ein diabetisches Fußgeschwür umfasst;
- die Wunde Fibrose nach einem Trauma, einer thermischen Verletzung oder einer Strahlenverbrennung umfasst; oder
- die Wunde eine Operationswunde umfasst.

6. Mit Kollagen I beschichtetes biokompatibles elektrogesponnenes Gerüst zur Verwendung nach Anspruch 5,
wobei die Operationswunde eine Wunde umfasst, die aus der folgenden Gruppe resultiert
ästhetische Operation, Hernienreparatur, Durareparatur, Reparatur des Orbitabodens, Brustrekonstruktion, urologische Reparatur, gynäkologische Reparatur und Kombinationen davon.

7. Mit Kollagen I beschichtetes biokompatibles elektrogesponnenes Gerüst zur Verwendung nach einem der Ansprüche 1-6, wobei das Gerüst mit einer oder mehreren zusätzlichen Verbindungen kombiniert wird, die aus der aus einer Narbenbildung verhindernden Verbindung, einer heilungsfördernden Verbindung und einer entzündungshemmenden Verbindung bestehenden Gruppe ausgewählt sind.

8. Mit Kollagen I beschichtetes biokompatibles elektrogesponnenes Gerüst zur Verwendung nach Anspruch 7, wobei die eine oder mehreren zusätzlichen Verbindungen aus der aus einem Statin, Losartan, mikroinhibitorischen RNAs (miRs), Peptiden und Antikörpern bestehenden Gruppe ausgewählt sind.

9. Mit Kollagen I beschichtetes biokompatibles elektrogesponnenes Gerüst zur Verwendung nach einem der Ansprüche 1-8, wobei das Gerüst ein 50:50-Gemisch von Poly(milchsäure) (PLA) und Poly(ε-Caprolacton) (PCL) umfasst.

10. Mit Kollagen I beschichtetes biokompatibles elektrogesponnenes Gerüst zur Verwendung nach einem der Ansprüche 1-9, wobei das Gerüst zu einer Dicke von ungefähr 50 µm bis ungefähr 5 mm elektrogesponnen wird.

11. Mit Kollagen I beschichtetes biokompatibles elektrogesponnenes Gerüst zur Verwendung nach einem der Ansprüche 1-10, wobei das Gerüst zu einer durchschnittlichen Porengröße von ungefähr 10 µm bis ungefähr 300 µm elektrogesponnen wird.

## Revendications

1. Échafaudage électrofilé biocompatible revêtu de collagène de type 1 destiné à être utilisé dans un procédé de prévention et/ou de réduction de la formation de cicatrice d'une plaie chez un sujet, le procédé comprenant l'implantation de l'échafaudage électrofilé biocompatible revêtu de collagène de type 1 dans la plaie du sujet dans le but de favoriser la formation de tissu de granulation et de prévenir la contraction de la plaie et de la cicatrice.

2. Échafaudage électrofilé biocompatible revêtu de collagène de type 1 destiné à être utilisé dans un procédé de prévention et/ou de réduction de la rétraction d'une cicatrice dans une plaie chez un sujet, le procédé comprenant l'implantation de l'échafaudage électrofilé biocompatible revêtu de collagène de type 1 dans la plaie du sujet dans le but de réduire au minimum la transmission de contrainte mécanique et/ou de réduire l'alignement de la MEC et l'inflammation, ce qui permet de prévenir et/ou de réduire la rétraction de la cicatrice.

3. Échafaudage électrofilé biocompatible revêtu de collagène de type 1 destiné à être utilisé selon la revendication 1 ou 2, l'échafaudage comprenant de la poly(l-lactide-co-ε-caprolactone) (PLCL).

4. Échafaudage électrofilé biocompatible revêtu de collagène de type 1 destiné à être utilisé selon l'une quelconque des revendications 1 à 3, l'échafaudage biocompatible revêtu de collagène de type 1 étant implanté dans une plaie ouverte ou sous un greffon de peau appliqué.

5. Échafaudage électrofilé biocompatible revêtu de collagène de type 1 destiné à être utilisé selon l'une quelconque des revendications 1 à 4, dans lequel :
- la plaie comprend une plaie chronique ;
- la plaie comprend un ulcère de stase veineuse ou un ulcère de pied diabétique ;
- la plaie comprend une fibrose consécutive à un traumatisme, une lésion thermique ou une brûlure par radiation ; ou
- la plaie comprend une plaie chirurgicale.

6. Échafaudage électrofilé biocompatible revêtu de collagène de type 1 destiné à être utilisé selon la revendication 5, dans lequel la plaie chirurgicale comprend une plaie obtenue du groupe consistant en chirurgie esthétique, réparation herniaire, réparation durale, réparation du plancher de l'orbite, reconstruction mammaire, réparation urologique, réparation gynécologique et des combinaisons de celles-ci.

7. Échafaudage électrofilé biocompatible revêtu de collagène de type 1 destiné à être utilisé selon l'une quelconque des revendications 1 à 6, l'échafaudage étant combiné à un ou plusieurs composés supplémentaires choisis dans le groupe constitué par un composé contre la formation de cicatrice, un composé favorisant la cicatrisation et un composé anti-inflammatoire.

8. Échafaudage électrofilé biocompatible revêtu de collagène de type 1 destiné à être utilisé selon la revendication 7, dans lequel le ou les composés supplémentaires sont choisis dans le groupe constitué par une statine, le losartan, des inhibiteurs de micro-ARN (miR), des peptides et des anticorps.

9. Échafaudage électrofilé biocompatible revêtu de collagène de type 1 destiné à être utilisé selon l'une quelconque des revendications 1 à 8, l'échafaudage comprenant un mélange à 50:50 d'acide polylactique (PLA) et de poly(ε-caprolactone) (PCL).

10. Échafaudage électrofilé biocompatible revêtu de collagène de type 1 destiné à être utilisé selon l'une quelconque des revendications 1 à 9, l'échafaudage étant électrofilé à une épaisseur d'environ 50 µm à environ 5 mm.

11. Échafaudage électrofilé biocompatible revêtu de collagène de type 1 destiné à être utilisé selon l'une quelconque des revendications 1 à 10, l'échafaudage étant électrofilé à des tailles de pores moyennes d'environ 10 µm à environ à 300 µm.
